# EUROPÄISCHE PATENTSCHRIFT

(11) **EP 3 116 561 B1**
(45) Veröffentlichungstag und Bekanntmachung des Hinweises auf die Patenterteilung: **12.01.2022**
(21) Anmeldenummer: 15709183.6
(22) Anmeldetag: 11.03.2015
(51) Int. Cl.: A61M 1/16

(54) **VORRICHTUNG ZUM BILANZIEREN ZWISCHEN EINEM ZUFLUSS UND EINEM ABFLUSS AUS EINER MEDIZINISCHEN BEHANDLUNGSVORRICHTUNG**
DEVICE FOR BALANCING BETWEEN AN INFLOW INTO AND AN OUTFLOW FROM A MEDICAL TREATMENT DEVICE
DISPOSITIF SERVANT À COMPTABILISER UN FLUX ENTRANT PAR RAPPORT À UN FLUX SORTANT D'UN DISPOSITIF DE TRAITEMENT MÉDICAL

(30) Priorität: 13.03.2014 DE 102014003619
(43) Veröffentlichungstag der Anmeldung: 18.01.2017
(73) Patentinhaber: Fresenius Medical Care Deutschland GmbH, 61352 Bad Homburg (DE)
(72) Erfinder: WIKTOR, Christoph, 63571 Gelnhausen (DE); PETERS, Arne, 61352 Bad Homburg (DE)
(74) Vertreter: Dreyhsig, Jörg
(86) Internationale Anmeldenummer: PCT/EP2015/055074
(87) Internationale Veröffentlichungsnummer: WO 2015/135989

(56) Entgegenhaltungen:
- DE-A1- 19 702 211
- DE-A1-102010 023 635
- US-A1- 2009 124 963
- US-A1- 2009 198 170
- None

## Beschreibung

### Technisches Gebiet

Die Erfindung betrifft das Gebiet der Bilanziervorrichtungen für eine medizinische Behandlungsvorrichtung, insbesondere zur Bilanzierung von Dialysierfüssigkeit zwischen einem Zufluss in einen Dialysator und einem Abfluss aus dem Dialysator.

### Hintergrund

Es sind verschiedene Arten von Behandlungsvorrichtungen bekannt, die über eine mit Flüssigkeit zu versorgende Behandlungseinheit verfügen. Zu den bekannten Behandlungsvorrichtungen gehören beispielsweise die Blutbehandlungsvorrichtungen. So sind in der Nierenersatztherapie verschiedene Blutreinigungsverfahren etabliert, bei denen das Blut extrakorporal von harnpflichtigen Blutinhaltsstoffen, das heißt Blutinhaltsstoffen, die bei einem Gesunden über die Nieren ausgeschieden werden, befreit wird.

Andere bekannte extrakorporale Blutbehandlungsvorrichtungen sind Plasmaseparationssysteme, sowie Adsorber für eine Leberunterstützungstherapie oder zur Behandlung einer Sepsis.

Bei der Hämodialyse findet ein diffusiver Stofftransport von in dem Blut enthaltenen harnpflichtigen Substanzen über eine semipermeable Membran in eine Dialysierflüssigkeit statt. Der Stofftransport findet über die semipermeable Wand eines Dialysators statt.

Der Dialysator weist eine an einen extrakorporalen Blutkreislauf angeschlossene Blutkammer und eine an einen Dialysierflüssigkeitskreislauf angeschlossene Dialysierflüssigkeitskammer auf. Blutkammer und Dialysierflüssigkeitskammer sind von der semipermeablen Membran getrennt. Um einen diffusiven Verlust von Elektrolyten, die im Blut verbleiben sollen, zu verhindern, enthält die Dialysierflüssigkeit eine bestimmte Zusammensetzung von Elektrolyten, in einer physiologischen Konzentration.

Zusätzlich findet bei der Dialyse über die semipermeable Membran ein Entzug von überschüssiger Flüssigkeit statt, der durch den Druckgradient an der semipermeablen Membran herbeigeführt wird.

Demgegenüber findet bei der Hämofiltration zusätzlich ein konvektiver Stofftransport über eine semipermeable Membran eines Filters statt, bei der ein Druckgradient an der Membran auch die treibende Kraft für den Stofftransport ist.

Zum Ausgleich eines Verlustes von erwünschten Blutinhaltsstoffen müssen die über die Membran verlorenen Elektrolyte durch eine Substituatflüssigkeit ersetzt werden. Eine Kombination eines konvektiven und eines diffusiven Transports wird als Hämodiafiltration bezeichnet. Im Kontext dieser Anmeldung soll unter dem Begriff der Dialyse oder einer Dialysebehandlung sowohl eine rein diffusive Dialyse als auch eine Hämodiafiltration verstanden werden.Wegen der großen Austauschmengen besteht bei den bekannten Verfahren und Vorrichtungen zur Dialyse die Notwendigkeit einer exakten Bilanzierung der dem Patienten entzogenen Flüssigkeit und der dem Patienten zugeführten Flüssigkeit über die gesamte Behandlungszeit. Zum Stand der Technik gehören gravimetrische und volumetrische Bilanziervorrichtungen.

Zur volumetrische Bilanzierung sind Bilanziervorrichtungen unter Verwendung von Bilanzkammern mit Trennelementen und Bilanziervorrichtungen unter Verwendung von Austauschgefäßen bekannt.

Eine Dialysevorrichtung mit einer Bilanziervorrichtung unter Verwendung von Bilanzkammern ist beispielsweise aus der DE 26 34 238 A1 bekannt. Die Bilanziervorrichtung der bekannten Hämodiafiltrationsvorrichtung weist einen volumenstarren Hohlkörper auf, der durch ein bewegliches Trennelement in eine Kammer für frisches und eine Kammer für verbrauchtes Dialysat unterteilt ist. Die Kammer für frisches Dialysat ist über eine Zuführleitung zu einer Quelle für frisches Dialysat und mit einer Abführleitung, über die das frische Dialysat dem Dialysator zugeführt wird, verbunden. Die Kammer für verbrauchtes Dialysat ist über eine Zuführleitung, aus der verbrauchtes Dialysat in die Kammer einströmen kann, und mit einer zu einem Abfluss für die Dialysierflüssigkeit führenden Abführleitung verbunden. Darüber hinaus sind Pumpen für die Förderung der frischen und der verbrauchten Dialysierflüssigkeit sowie eine Steuereinheit vorgesehen, die ein wechselseitiges Befüllen der beiden Kammern erlaubt. Hierbei beruht die Bilanziergenauigkeit darauf, dass die Kammer für verbrauchtes Dialysat und die Kammer für frisches Dialysat das gleiche Volumen aufweisen. Bilanzkammern werden daher üblicherweise mit einer geringen Fertigungstoleranz aus Materialien mit eine guten Volumenkonstanz hergestellt.

Eine weitere Dialysevorrichtung mit einer Bilanziervorrichtung unter Verwendung von Bilanzkammern ist aus der DE 10 2010 023635 A1 bekannt.

Ein alternatives Verfahren zur volumetrischen Bilanzierung beruht auf der Verwendung von Austauschgefäßen. Hierbei ist ein Austauschgefäß über fluidführende Leitungen mit einer Quelle für frische Dialysierflüsigkeit, mit einem Abfluss für die verbrauchte Dialysierflüssigkeit, mit einem Abfluss in den Dialysator und mit einem Zufluss von dem Dialysator verbindbar. In den fluidführenden Leitungen sind Absperrorgane oder Ventile vorgesehen, die derart ansteuerbar sind, dass in einem ersten Arbeitstakt ein erster Flüssigkeitskreislauf gebildet wird, der die Flüssigkeitsquelle über das Austauschgefäß mit dem Abfluss verbindet und in einem zweiten Arbeitstakt ein Flüssigkeitskreislauf gebildet wird, der den Abfluss des Dialysators über das Austauschgefäß mit dem Zufluss in den Dialysator verbindet.

So strömt in dem ersten Arbeitstakt verbrauchte Dialysierflüssigkeit aus dem Austauschgefäß in den Abfluss und wird durch frische Dialysierflüssigkeit aus der Dialysierflüssigkeitsquelle ersetzt. In dem zweiten Arbeitstakt strömt die frische Dialysierflüssigkeit aus dem Austauschgefäß in den Dialysator und wird durch verbrauchte Flüssigkeit aus dem Dialysator ersetzt.

In dem zweiten Arbeitstakt entspricht die aus dem Dialysator abfließende der in den Dialysator einströmenden Flüßigkeitsmenge, und es wird so eine Bilanzierung gewährleistet.

Der einfache Aufbau eines Austauschgefäßes erlaubt die Ausführung des Dialysierflüssigkeitskreislaufs oder Teile des Dialysierflüssigkeitskreislaufs als Einmalartikel. Solche Einmalartikel können vorzugsweise aus PVC oder einem anderen Kunststoff hergestellt werden.

Die Erfinder der vorliegenden Anmeldung haben erkannt, dass es in diesem Fall zu einer Fehlbilanzierung kommen kann, wenn auf Grund des verwendeten Materials der Austauschkammer eine Volumenkonstanz bei unterschiedlichen Arbeitsdrücken nicht gegeben ist.

Die Erfinder der vorliegenden Anmeldung haben weiterhin erkannt, dass durch eine Kompression von in der Dialysierflüssigkeit vorhandenen Gasblasen eine Konstanz der Flüssigkeitsmengen nicht gegeben ist, wenn sich der Arbeitsdruck ändert.

Es ist daher die Aufgabe der vorliegenden Erfindung, eine Bilanziervorrichtung zur Bilanzierung mit erhöhter Bilanziergenauigkeit anzugeben.

### Zusammenfassung

Diese Aufgabe wird durch eine Bilanziervorrichtung nach Anspruch 1 gelöst. Vorteilhafte Weiterbildungen sind in den abhängigen Ansprüchen angegeben.

Im Einklang mit der vorliegenden Offenbarung wird eine Bilanziervorrichtung zum Bilanzieren zwischen einem Zufluss in eine Blutbehandlungseinheit einer medizinischen Behandlungsvorrichtung und einem Abfluss aus der Blutbehandlungseinheit, bereitgestellt.

Die medizinische Behandlungsvorrichtung kann als eine Hämodialysevorrichtung mit einem Dialysator als Blutbehandlungseinheit oder als eine Apheresevorrichtung mit einer einen Adsorber umfassenden Blutbehandlungseinheit ausgebildet sein.

Die Bilanziereinheit weist mindestens ein Austauschgefäß auf, eine zu dem Austauschgefäß führende erste Zuleitung zum Zuführen von Flüssigkeit aus einer Flüssigkeitsquelle, insbesondere aus einer Dialysierflüssigkeitsquelle, in das Austauschgefäß, und eine von dem Austauschgefäß abgehende erste Abflussleitung in zum Abführen der Flüssigkeit aus dem Austauschgefäß in einen Ablauf.

Darüber hinaus weist die Bilanziereinheit eine von dem Austauschgefäß abgehende zweite Abflussleitung zum Abführen von Flüssigkeit aus dem Austauschgefäß in die Behandlungseinheit und eine zu dem Austauschgefäß führende zweite Zuleitung zum Zuführen von Flüssigkeit aus der Behandlungseinheit in das Austauschgefäß auf.

Die Bilanziereinheit umfasst weiterhin Mittel zum Fördern von Flüssigkeit in das und/ oder aus dem Austauschgefäß und Mittel zum Unterbrechen des Zuflusses von Flüssigkeit in das Austauschgefäß und/ oder Abflusses von Flüssigkeit aus dem Austauschgefäß sowie eine Steuereinheit zum Ansteuern der Mittel zum Fördern von Flüssigkeit und der Mittel zum Unterbrechen des Zuflusses und/ oder Abflusses von Flüssigkeit.

Die Steuereinheit ist ausgebildet, die Mittel zum Fördern der Flüssigkeit und/ oder die Mittel zum Unterbrechen des Zuflusses derart anzusteuern, dass in einem ersten Arbeitstakt von aufeinanderfolgenden Arbeitstakten ein erster Fluidkreislauf gebildet wird, in dem verbrauchte Flüssigkeit aus dem Austauschgefäß in den Abfluss strömt und durch frische Flüssigkeit aus der Flüssigkeitsquelle ersetzt wird und in einem zweiten Arbeitstakt der aufeinanderfolgenden Arbeitstakte ein zweiter Fluidkreislauf gebildet wird, in dem frische Flüssigkeit aus dem Austauschgefäß in die Behandlungseinheit strömt und durch verbrauchte Flüssigkeit aus der Behandlungseinheit ersetzt wird.

Mit anderen Worten wird in dem ersten Arbeitstakt ein Flüssigkeitskreislauf gebildet, der die Flüssigkeitsquelle über die Behandlungseinheit mit dem Abfluss verbindet, und in einem zweiten Arbeitstakt wird ein Flüssigkeitskreislauf gebildet, der den Abfluss der Blutbehandlungseinheit über das Austauschgefäß mit dem Zufluss in die Blutbehandlungseinheit verbindet.

Die Bilanziervorrichtung weist weiterhin einen mit der Steuereinheit verbundenen Druckaufnehmer zum Aufnehmen des Drucks in dem Austauschgefäß während des ersten, des zweiten Arbeitstakts und während eines dritten Arbeitstakts auf.

Die Steuereinheit ist weiterhin ausgebildet, das Mittel zum Fördern von Flüssigkeit in das und/ oder aus dem Austauschgefäß und/oder die Mittel zum Unterbrechen des Zuflusses von Flüssigkeit in das Austauschgefäß und/ oder des Abflusses von Flüssigkeit aus dem Austauschgefäß im Hinblick auf einen Druckausgleich zwischen dem ersten und dem zweiten Arbeitstakt anzusteuern.

Dies kann geschehen, indem in einem dritten Arbeitstakt die erste und die zweite Abflussleitung abgesperrt werden und der Druck in dem Austauschgefäß auf den höheren der beiden Drücke gebracht wird, oder die erste und die zweite Zuleitung abgesperrt werden und der Druck in dem Austauschgefäß auf den niedrigeren der beiden Drücke gebracht wird.

In alternativen, illustrativen, nicht erfindungsgemäßen Ausführungsformen ist ein dritter Arbeitstakt nicht erforderlich. In einer dieser Ausführungsformen ist das Mittel zum Fördern von Flüssigkeit als eine druckgesteuerte Pumpe ausgebildet, die derart angesteuert wird, dass in dem ersten Arbeitstakt der Druck in dem Austauschgefäß im Wesentlichen gleich dem Druck in dem Austauschgefäß in dem zweiten Arbeitstakt ist, wobei diese Bedingung insbesondere zum Ende des jeweiligen Arbeistakts eingehalten wird. Alternativ oder ergänzend dazu ist eine Drossel zur Kontrolle des Flusses durch die erste und/ oder die zweite Abflussleitung vorgesehen, wobei die Steuereinheit weiterhin zum Ansteuern der Drossel ausgebildet ist, so dass in dem ersten Arbeitstakt der Druck in dem Austauschgefäß im Wesentlichen gleich dem Druck in dem Austauschgefäß in dem zweiten Arbeitstakt ist, wobei diese Bedingung insbesondere zum Ende des jeweiligen Arbeitstakts eingehalten wird.

In einer Weiterbildung der beschriebenen Bilanziervorrichtungen ist das Mittel zum Fördern von Flüssigkeit in die und/ oder aus dem Austauschgefäß derart mit dem ersten und zweiten Zufluss oder mit dem ersten und zweiten Abfluss durch eine fluidführende Leitung verbindbar, dass das Mittel zum Fördern von Flüssigkeit während des ersten Arbeitstakts und während des zweiten Arbeitstakts Flüssigkeit fördern kann. Ein einziges Mittel zum Fördern der frischen und /oder gebrauchten Flüssigkeit reicht somit aus.

In einer Weiterbildung der Bilanziervorrichtung ist die Steuereinheit eingerichtet, die Mittel zum Fördern von Flüssigkeit in die und/ oder aus dem Austauschgefäß unter Verwendung eines Messsignals des Druckaufnehmers anzusteuern.

In einer vorteilhaften Ausführungsform ist das oder die Mittel zum Fördern von Flüssigkeit in die und/ oder aus dem Austauschgefäß eine druckgesteuerte Pumpe, das heißt eine Pumpe, die im Hinblick auf einen bestimmten Förderdruck einstellbar ist. In einer Weiterbildung dieser Ausführungsform ist die druckgesteuerte Pumpe eine Impellerpumpe (z.B. eine Zentrifugalpumpe) oder eine Membranpumpe.

Als Druckaufnehmer kann ein gasgefülltes Ausgleichsgefäß vorgesehen sein, das in Fluidverbindung mit dem Austauschgefäß steht, wobei ein Messmittel zum Bestimmen des Flüssigkeitsniveaus in dem Ausgleichsgefäß vorgesehen ist, und wobei das Flüssigkeitsniveau ein Maß für den Duck in dem Austauschgefäß angibt.

In einer Ausführungsform der Bilanziervorrichtung ist das Austauschgefäß bei einer Änderung des Drucks in dem Austauschgefäß nicht volumenkonstant.

In einer weiteren Ausführungsform der Bilanziervorrichtung ist das Austauschgefäß als Einwegartikel oder als Teil eines Einwegartikels ausgeführt.

Zusätzlich kann mindestens eine der Fluidleitungen, die einen Zufluss in das oder einen Abfluss aus dem Austauschgefäß bilden, als Teil eines Einwegartikels, vorzugsweise als Teil des das Austauschgefäß enthaltenden Einwegartikels ausgeführt sein. Hierbei können eines oder mehrere der Absperrorgane oder Ventile durch Klemmen an den Fluidleitungen ausgeführt sein.

Die beschriebene Bilanziervorrichtung kann Teil einer medizinischen Behandlungsvorrichtung, insbesondere Teil einer Dialysevorrichtung sein.

Im Einklang mit der vorliegenden Offenbarung wird weiterhin ein illustratives, nicht beanspruchtes Verfahren zum Bilanzieren zwischen einem Zufluss in eine Blutbehandlungseinheit einer medizinischen Behandlungsvorrichtung und einem Abfluss aus der Blutbehandlungseinheit beschrieben.

Bei dem illustrativen, nicht beanspruchten Verfahren sind aufeinanderfolgende erste und zweite Arbeitstakte vorgesehen, wobei in dem ersten Arbeitstakt ein erster Fluidkreislauf gebildet wird, in dem verbrauchte Flüssigkeit aus einem Austauschgefäß über eine erste Abflussleitung in einen Abfluss strömt und frische Flüssigkeit aus einer Dilaysierflüssigkeitsquelle über eine erste Zuleitung in das Austauschgefäß nachströmt. In dem zweiten Arbeitstakt wird ein zweiter Fluidkreislauf gebildet, in dem frische Flüssigkeit aus dem Austauschgefäß über eine zweite Abflussleitung in die Blutbehandlungseinheit strömt und über eine zweite Zuleitung durch verbrauchte Flüssigkeit aus der Blutbehandlungseinheit ersetzt wird. Der Druck in dem Austauschgefäß wird während des ersten und während des zweiten Arbeitstakts gemessen. Weiterhin werden Mittel zum Fördern von Flüssigkeit in das und/ oder aus dem Austauschgefäß und/ oder Mittel zum Unterbrechen des Zuflusses von Flüssigkeit in das Austauschgefäß und/ oder Abflusses von Flüssigkeit aus dem Austauschgefäß im Hinblick auf einen Druckausgleich zwischen dem ersten und dem zweiten Arbeitstakt angesteuert.

Dies kann geschehen, indem in einem dritten Arbeitstakt die erste und die zweite Abflussleitung abgesperrt werden und der Druck in dem Austauschgefäß auf den höheren der beiden Drücke gebracht wird, oder die erste und die zweite Zuleitung abgesperrt werden und der Druck in dem Austauschgefäß auf den niedrigeren der beiden Drücke gebracht wird.

In alternativen, illustrativen, nicht erfindungsgemäßen Ausführungsformen ist ein dritter Arbeitstakt nicht erforderlich. In einer dieser Ausführungsformen ist das Mittel zum Fördern von Flüssigkeit als eine druckgesteuerte Pumpe ausgebildet. Die druckgesteuerte Pumpe wird unter Verwendung des Drucksignals so angesteuert, das während des ersten Arbeitstakts der Druck in dem Austauschgefäß im Wesentlichen gleich dem Druck in dem Austauschgefäß während des zweiten Arbeitstakts ist, wobei diese Bedingung insbesondere zum Ende des jeweiligen Arbeistakts eingehalten wird.

Alternativ oder ergänzend dazu ist eine Drossel zur Kontrolle des Flusses durch die erste oder die zweite Abflussleitung vorgesehen, In dieser Ausführungsform wird der gemessene Druck verwendet, um die Drossel oder zusätzlich die Drossel so anzusteuern, dass mindestens in einem Teil des ersten Arbeitstakts der Druck in der Austauschgefäß im Wesentlichen gleich dem Druck in der Austauschgefäß mindestens in einem Teil des zweiten Arbeitstakts ist, wobei diese Bedingung insbesondere zum Ende des jeweiligen Arbeitstakts einzuhalten ist.

Die Verfahren zum Bilanzieren sind denselben oder entsprechenden Modifikationen zugänglich, wie sie im Zusammenhang mit der entsprechenden Bilanziervorrichtung beschrieben wurden.

### Kurze Beschreibung der Zeichnungen

Figur 1 zeigt eine Hämodialysevorrichtung mit einem Dialysator und mit einer Bilanziervorrichtung zum Bilanzieren zwischen einem Zufluss aus dem Dialysator und dem Abfluss aus dem Dialysator.
Figur 2 zeigt die Bilanziervorichtung von Figur 1 in einem ersten Arbeitstakt des Arbeitszyklus.
Figur 3 zeigt die Bilanziervorrichtung von Figur 1 in einem zweiten Arbeitstakt des Arbeitszyklus.
Figur 4 zeigt die Bilanziervorrichtung von Figur 1 in einem dritten Arbeitstakt.
Figuren 5 - 7 zeigen weitere Arbeitstakte einer Bilanziervorrichtung.
Figur 8 zeigt eine weitere Ausführungsform einer Hämodialysevorrichtung mit einer Bilanziereinrichtung.
Figur 9 zeigt eine alternative Ausführungsform einer Hämodialysevorrichtung mit einer Bilanziereinrichtung.
Figur 10 zeigt eine alternative Ausführungsform einer Bilanziervorrichtung.

### Detaillierte Beschreibung der Zeichnungen

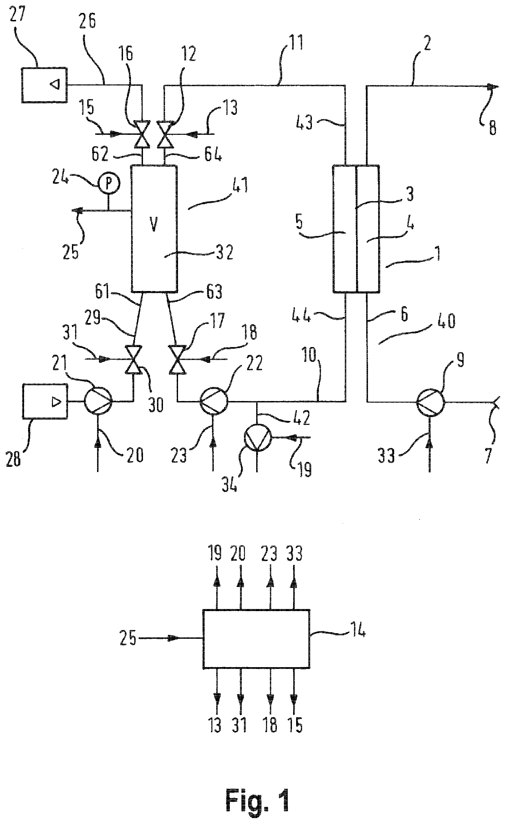

Figur 1 zeigt ein Beispiel einer Hämodialysevorrichtung als Blutbehandlungsvorrichtung, die über einen Dialysator 1 als Blutbehandlungseinheit verfügt. Fig. 1 zeigt in stark vereinfachter schematischer Darstellung die wesentlichen Komponenten der Hämodialysevorrichtung einschließlich einer Bilanziervorrichtung 41. Die Dialysevorrichtung weist einen Dialysator 1 auf, der durch eine semipermeable Membran 3 in eine Blutkammer 4 und eine Dialysierflüssigkeitskammer 5 unterteilt ist. Die Bilanziervorrichtung 41 dient dem Bilanzieren zwischen einem Zufluss aus dem Dialysator 1 und dem Abfluss aus dem Dialysator 1 und ist ein Bestandteil der Dialysevorrichtung. Von dem zu behandelnden Patienten wird Blut über einen Zugang 7 entnommen und über eine Blutzuführleitung 6 in die eine Blutpumpe 9 geschaltet ist, zu der Blutkammer 4 des Dialysators 1 geführt. Von der Blutkammer 4 des Dialysators geht eine Blutabführleitung 2 ab, die das dialysierte Blut über einen Zugang 8 an den Patienten zurückgibt. Während der Blutbehandlung strömt das Blut des Patienten in dem extrakorporalen Blutkreislauf 40 durch die Blutkammer 4 des Dialysators 1.

Der Dialysator 1 wird mit Dialysierflüssigkeit versorgt, die durch die Dialysierflüssigkeitskammer 5 des Dialysators 1 strömt.

Zum Bilanzieren zwischen der der Dialysierflüssigkeitskammer 5 zugeführten und aus der Dialysierflüssigkeitskammer 5 abgeführten Dialysierflüssigkeit dient eine Bilanziereinheit 41, die über ein Austauschgefäß 32 verfügt. Das Austauschgefäß 32 weist einen ersten Einlass 61 für frische Dialysierflüssigkeit sowie einen ersten Auslass 62 für verbrauchte Dialysierflüssigkeit auf. Ein zweiter Auslass 64 des Austauschgefäßes 32 führt über die Leitung 11 zu einem Einlass 43 der Dialysierflüssigkeitskammer 5. Über einen zweiten Einlass 63 des Austauschgefäßes 32 kann verbrauchte Dialysierflüssigkeit von der Dialyserflüssigkeitskammer 5 in das Austauschgefäß 32 strömen.

Die Dialysierflüssigkeit wird in einer Dialysierflüssigkeitsquelle 28 bereitgestellt, bei der es sich um einen Kanister oder einen Beutel handeln kann. In einer alternativen Ausführungsform der Dialysierflüssigkeitsquelle 28 wird die Dialysierflüssigkeit auch während der Behandlung, d.h. online aus entsprechenden Konzentraten und Reinwasser (RO-Wasser) hergestellt.

Von der Dialysierflüssigkeitsquelle 28 geht eine erste Zuleitung 29 ab, die zu dem ersten Einlass 61 des Austauschgefäßes 32 führt. Von dem ersten Auslass 62 des Austauschgefäßes geht eine erste Abflussleitung 26 ab, die zu einem Ablauf oder Abfluss 27 führt. In die erste Zuleitung 29 ist eine erste Dialysierflüssigkeitspumpe 21 geschaltet, die vorteilhaft als druckgesteuerte Pumpe, d.h. eine Pumpe, die im Hinblick auf ihren Förderdruck angesteuert wird, ausgeführt ist, beispielsweise als Impellerpumpe oder als Membranpumpe. Die erste Dialysierflüssigkeitspumpe 21 fördert die frische Dialysierflüssigkeit aus der Dialysierfiüssigkeitsquelle 28 in das Austauschgefäß 32.

Von dem zweiten Auslass 64 des Austauschgefäßes 32 geht eine zweite Abflussleitung 11 ab, die zu einem Einlass 43 der Dialysierflüssigkeitskammer 5 des Dialysators 1 führt. Von dem Auslass 44 der Dialysierflüssigkeitskammer 5 des Dialysators 1 geht eine zweite Zuleitung 10 ab, die zu dem zweiten Einlass 63 des Austauschgefäßes 32 führt. In die zweite Zuleitung 10 ist eine zweite Dialysierflüssigkeitspumpe 22, die vorteilhaft als druckgesteuerte Pumpe, beispielsweise als Impellerpumpe oder als Membranpumpe ausgeführt ist, geschaltet, die verbrauchte Dialysierflüssigkeit aus der Dialysierflüssigkeitskammer 5 in das Austauschgefäß 32 fördert. Ein Druckaufnehmer, ein Druckmesser oder eine Druckmessdose 24 ist zur Messung des Drucks in dem Austauschgefäß vorgesehen.

Die Zu- und Abflussleitungen 10, 11, 29, 26 sind vorteilhaft als Schlauchleitungen ausgeführt.

Das Austauschgefäß ist vorteilhaft als Wegwerfartikel oder als Teil eines Wegwerfartikels ausgeführt, das besonders vorteilhaft noch die Zu- und Abflussleitungen umfasst.

Von der Zuleitung 10 geht eine Ultrafiltrationsleitung 42 ab, in die eine Ultrafiltrationspumpe 34 geschaltet ist. Soweit die Bilanziervorrichtung 41 für eine ausgeglichene Bilanz zwischen frischer und verbrauchter Dialysierflüssigkeit sorgt, bildet die über die Ultrafiltrationspumpe 19 entzogene Flüssigkeitsmenge ein Maß für die dem extrakorporalen Blutkreislauf über die semipermeable Membran 3 des Dialysators 1 entzogene Flüssigkeitsmenge.

Der Zufluss zu dem Austauschgefäß 32 kann durch Ventile 30, 17 der ersten 29 und zweiten 10 Zuleitung unterbrochen werden und ist so steuerbar. Anderseits kann der Abfluss aus dem Austauschgefäß durch Ventile 16, 12 in der ersten 26 und zweiten 11 Abflussleitung unterbrochen werden.

Die Ventile 30, 17, 16, 12 sind über Steuerleitungen 31, 18, 15, 13 mit einer Steuereinheit 14 verbunden, und durch diese ansteuerbar. Die Ventile 30, 17, 16 und 12 können als elektromagnetisch zu betätigende Schlauchklemmen ausgeführt sein. Die erste 21 und die zweite 22 Dialysierflüssigkeitspumpe sind außerdem über Steuerleitungen 20, 23 mit der Steuereinheit 14 verbunden und durch diese ansteuerbar. Der Druckaufnehmer, der Druckmesser oder die Druckmessdose 24 ist weiterhin über eine Datenleitung 25 mit der Steuereinheit 14 verbunden und stellt dieser so Messwerte des Drucks in dem Austauschgefäß 32 zur Verfügung. Die Steuereinheit 14 ist bei dem vorliegenden Beispiel Bestandteil der zentralen Steuereinheit der Dialysevorrichtung. Die zentrale Steuereinheit 14 der Dialysevorrichtung ist weiterhin über eine Steuerleitung 19 mit der Ultrafiltrationspumpe 34 sowie über eine Steuerleitung 33 auch mit der Blutpumpe 9 verbunden.

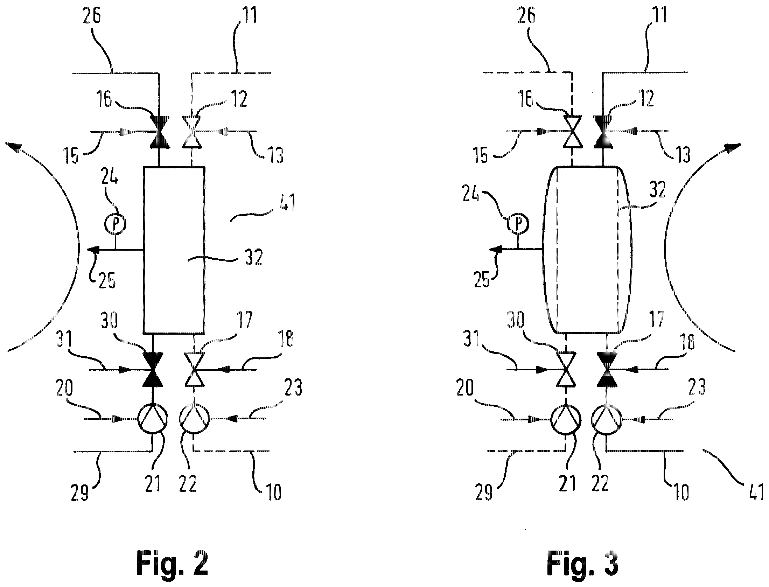

Die Steuereinheit 14 steuert die Ventile 30, 17, 16, 12 sowie die erste 21 und zweite 22 Dialysierflüssigkeitspumpe, so dass abwechselnd in einem ersten Arbeitstakt von aufeinanderfolgenden Arbeitstakten ein erster Fluidkreislauf gebildet wird, in dem verbrauchte Flüssigkeit aus dem Austauschgefäß 32 in den Abfluss 27 strömt und durch frische Flüssigkeit aus der Dialysierflüssigkeitsquelle 28 ersetzt wird und in einem zweiten Arbeitstakt der aufeinanderfolgenden Arbeitstakte ein zweiter Fluidkreislauf gebildet wird, in dem die frische Flüssigkeit aus dem Austauschgefäß 32 in den Dialysator strömt und durch verbrauchte Flüssigkeit aus dem Dialysator ersetzt wird, wie nachfolgend an Hand der Figuren 2 und 3 beschrieben wird.

Figur 2 zeigt die bereits im Zusammenhang mit der Figur 1 beschriebene Bilanziervorrichtung 41 während eines ersten Arbeitstakts. Mit entsprechenden Bezugszeichen versehene Elemente entsprechen denen der Figur 1, auf die Bezug genommen wird, an Stelle einer Wiederholung.

Der in der Figur 2 dargestellte erste Arbeitstakt dient dem Befüllen des Austauschgefäßes mit frischer Dialysierflüssigkeit aus der Dialysierflüssigkeitsquelle 28. In dem in Figur 2 gezeigten erste Arbeitstakt fördert die Dialysierflüssigkeitspumpe 21 frische Dialysierflüssigkeit von der Dialysierflüssigkeitsquelle 28 durch die erste Zuleitung 29 in das Austauschgefäß 32. Dort verdrängt sie die zu Beginn des ersten Arbeitstakts noch in dem Austauschgefäß befindliche verbrauchte Dialysierflüssigkeit, die durch die erste Abflussleitung 26 in den Abfluss 27 geleitet wird. Die in der Figur 2 dargestellte Pfeilrichtung deutet die Flussrichtung der Dialysierflüssigkeit während des ersten Arbeitstakts an. Während des ersten Arbeitstakts werden das Ventil 30 sowie das Ventil 16 offen gehalten und das Ventil 17 sowie das Ventil 12 geschlossen gehalten. So wird ein von der Dialysierflüssigkeitsquelle 28 über das Austauschgefäß zu dem Abfluss führender erster Dialysierflüssigkeitskreislauf gebildet, in dem Dialysierflüssigkeit durch die Dialysierflüssigkeitspumpe 20 umgewälzt wird. Soweit ein konstantes Volumen des Austauschgefäßes während des ersten Arbeitstakts gegeben ist, entspricht so die von der Dialysierflüssigkeitsquelle 28 in das Austauschgefäß einströmende Flüssigkeitsmenge der in den Abfluss 27 abfließenden Flüssigkeitsmenge. Der erste Arbeitstakt wird vorteilhaft so lange aufrechterhalten, dass davon ausgegangen werden kann, dass zum Ende des ersten Arbeitstakts die verbrauchte Dialysierflüssigkeit im Austauschgefäß 32 vollständig oder fast vollständig von der frischen Dialysierflüssgkeit verdrängt ist.

Dem ersten Arbeitstakt nachfolgend schließt sich ein zweiter Arbeitstakt an, der dem Befüllen der Dialysierflüssigkeitskammer 5 des Dialysators 1 mit frischer Dialysierflüssigkeit aus dem Austauschgefäß dient. In dem in Figur 3 dargestellten zweiten Arbeitstakt fördert die Dialysierflüssigkeitspumpe 22 verbrauchte Dialysierflüssigkeit aus der Dialysierflüssigkeitskammer 5 durch die zweite Zuleitung 10 in das Austauschgefäß 32. Dort verdrängt sie die zu Beginn des zweiten Arbeitstakts in dem Austauschgefäß befindliche frische Dialysierflüssigkeit, die durch die zweite Abflussleitung 11 der Dialysierflüssigkeitskammer 5 zugeführt wird. Die in der Figur 3 dargestellte Pfeilrichtung deutet die Flussrichtung der Dialysierflüssigkeit während des zweiten Arbeitstakts an. Während des zweiten Arbeitstakts werden das Ventil 17 sowie das Ventil 12 offen gehalten und das Ventil 30 sowie das Ventil 16 geschlossen gehalten. So wird ein zweiter Dialysierflüssigkeitskreislauf gebildet, in dem die Dialysierflüssigkeitskammer 5 mit dem Austauschgefäß 32 verbunden wird, und in dem Dialysierflüssigkeit durch die Dialysierflüssigkeitspumpe 22 umgewälzt wird.

Der erste Arbeitstakt wird vorteilhaft so lange aufrechterhalten, dass davon ausgegangen werden kann, dass zum Ende des zweiten Arbeitstakts die frische Dialysierflüssigkeit in die Dialysierflüssigkeitskammer 5 geströmt ist und dort die verbrauchte Dialysierflüssigkeit vollständig oder fast vollständig von der frischen Dialysierflüssgkeit verdrängt ist.

Der Druck in dem Teil des Dialysierflüssigkeitskreislaufs, der während des ersten Arbeitstakts mit dem Austauschgefäß 32 verbundenen ist, kann sich von dem Druck in dem Teil des Dialysierflüssigkeitslaufs unterscheiden, der während des zweiten Arbeitstakts mit dem Austauschgefäß 32 verbundenen ist. Insbesondere kann sich der Druck in dem Austauschgefäß 32 während des ersten Arbeitstakts von dem Druck in dem Austauschgefäß 32 während des zweiten Arbeitstakts unterscheiden. Wenn eine Volumenkonstanz des Austauschgefäßes bei unterschiedlichen Drücken nicht gewährleistet ist, kann sich entsprechend das Volumen des Austauschgefäßes 32 während des ersten Arbeitstaktes von dem Volumen des Austauschgefäßes während des zweiten Arbeitstakts unterscheiden. Eine Situation, bei der der Druck in dem Austauschgefäß 32 während des zweiten Arbeitstakts größer ist als während des ersten Arbeitstakts ist durch das in Figur 3 bauchiger dargestellte Austauschgefäß angedeutet.

Wenn eine Volumenkonstanz des Austauschgefäßes zwischen dem ersten und dem zweiten Arbeitstakt nicht gegeben ist, kann es zu einer Fehlbilanzierung beim Übergang zwischen dem ersten und dem zweiten Arbeitstakt kommen, wenn keine zusätzlichen Maßnahmen getroffen werden.

Um eine solchen Fehlbilanzierung auszugleichen oder zu vermeiden, wird der Druck in dem Austauschgefäß 32 während des ersten und/ oder während des zweiten Arbeitstaktstakts von dem Druckaufnehmer, dem Druckmesser oder der Druckmessdose 24 bestimmt, und über die Datenleitung 25 an die Steuereinheit 14 übermittelt.

Die Steuereinheit 14 ist weiterhin ausgebildet, die Dialysierflüssigkeitspumpe 21, die Dialysierflüssigkeitspumpe 22, das Ventil 12, und/ oder das Ventil 16 im Hinblick auf einen Druckausgleich zwischen dem ersten und dem zweiten Arbeitstakt anzusteuern.

Erfindungsgemäß wird dazu ein dritter Arbeitstakt eingeführt, in dem das Ventil 16 und das Ventil 13 abgesperrt oder geschlossen werden oder geschlossen gehalten werden und der Druck in dem Austauschgefäß auf einen zuvor gemessenen Druck gebracht wird, wobei der zuvor gemessene Druck dem höheren der Drücke in dem Austauschgefäß während des ersten Arbeitstakts und während des zweiten Arbeitstakts entspricht.

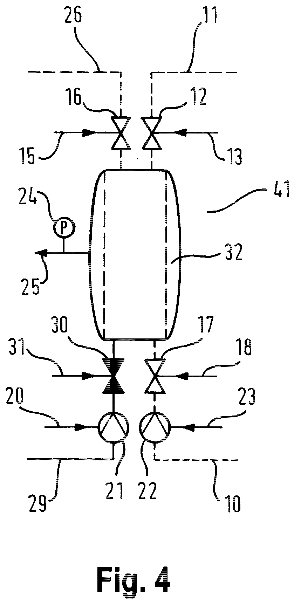

Figur 4 zeigt die den Figuren 2 und 3 entsprechende Situation, bei der in dem Austauschgefäß während des zweiten Arbeitstakts ein höherer Druck als während des ersten Arbeitstakts herrscht.

In diesem Fall wird in dem dritten Arbeitstakt der Zufluss von dem Teil des Dialysierflüssigkeitskreislaufs, der in dem zweiten Arbeitstakt verbunden war abgesperrt, das heißt das Ventil 17 wird abgesperrt oder geschlossen oder geschlossen gehalten und die Dialysierflüssigkeitspumpe 22 abgestellt. Weiterhin wird der Zufluss von dem Teil des Dialysierflüssigkeitskreislaufs, der in dem ersten Arbeitstakt verbunden war, geöffnet, das heißt das Ventil 30 wird geöffnet oder offen gehalten. Durch entsprechendes Ansteuern der druckgesteuerten Dialysierflüssigkeitspumpe 21 (D.h. der im ersten Arbeitstakt im ersten Dialysierflüssigkeitskreislauf befindlichen Dialysierflüssigkeitspumpe) stellt sich während des dritten Arbeitstakts in dem Austauschgefäß 32 ein Druck ein, der dem Druck während des zweiten Arbeitstakts entspricht, also demjenigen Arbeitstakt, in dem ein höherer Druck in dem Austauschgefäß herrschte.

Der dritte Arbeitstakt kann sich in der in der zeitlichen Abfolge der Arbeitstakte an den ersten Arbeitstakt und/ oder an den zweiten Arbeitstakt anschließen.

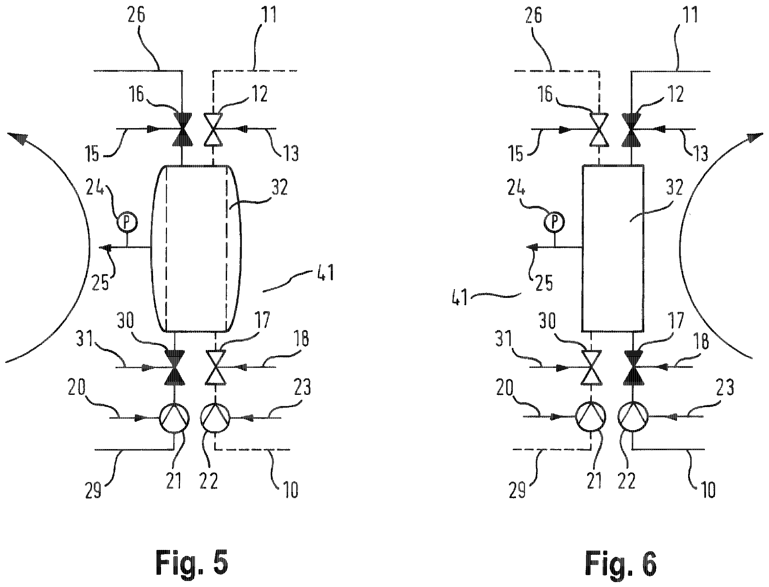

In den Figuren 5 und 6 ist die Situation dargestellt, bei der in dem Austauschgefäß während des ersten Arbeitstakts ein höherer Druck als während des zweiten Arbeitstakts herrscht, wobei der höhere Arbeitsdruck während des ersten Arbeiststakts durch die stärker bauchige Form des Austauschgefäßes 32 augedrückt werden soll.

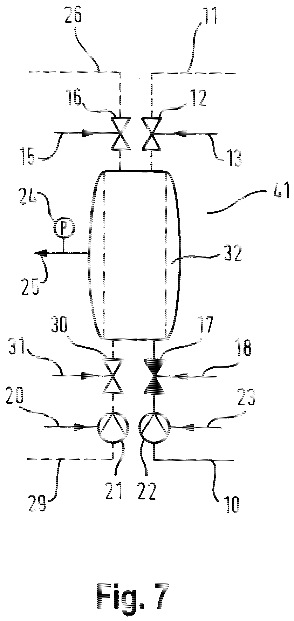

In diesem Fall wird in dem in der Figur 7 dargestellten dritten Arbeitstakt der Zufluss von dem Teil des Dialysierflüssigkeitskreislaufs, der in dem ersten Arbeitstakt verbunden war, abgesperrt, das heißt das Ventil 30 wird abgesperrt oder geschlossen oder geschlossen gehalten und die Dialysierflüssigkeitspumpe 21 abgestellt. Weiterhin wird der Zufluss von dem Teil des Dialysierflüssigkeitskreislaufs, der in dem zweiten Arbeitstakt verbunden war, geöffnet, das heißt das Ventil 30 wird geöffnet oder offen gehalten. Durch entsprechendes Ansteuern der druckgesteuerten Pumpe 22 (D.h. der im zweiten Arbeitstakt im zweiten Dialysierflüssigkeitskreislauf befindlichen Dialysierflüssigkeitspumpe) stellt sich während des dritten Arbeitstakts in dem Austauschgefäß 32 ein Druck ein, der dem Druck in während des ersten Arbeitstakts entspricht, d.h. desjenigen Arbeitstakts, in dem ein höherer Druck in dem Austauschgefäß herrschte.

Wenn durch die Dimensionierung des Teils des Dialysierflüssigkeitskreislaufs, der in dem ersten Arbeitstakt mit dem Austauschgefäß verbunden ist und des Teils des Dialysierflüssigkeitskreislaufs, der in dem zweiten Arbeitstakt mit dem Austauschgefäß 32 verbunden ist, feststeht, in welchem der Arbeitstakte im Normalbetrieb ein höherer Druck in dem Austauschgefäß 32 herrschen wird, kann es ausreichend sein, wenn in diesem Arbeitstakt der Druck in dem Ausgleichsgefäß bestimmt wird.

Andererseits können sich die Druckverhältnisse in dem Teil des Dialysierflüssigkeitskreislaufs, der die Dialysierflüssigkeitskammer enthält, während der Dialysbehandlung ändern, und es kann nicht von vornherein feststehen, in welchem Teil des Dialysierflüssigkeitskreislaufs der höhere Druck herrscht. In diesem Fall kann es sinnvoll sein, den Druck in dem Austauschgefäß sowohl in dem ersten als auch in dem zweiten Arbeitstakt zu überwachen, um festzulegen, in welcher Art ein Druckausgleich mittels des dritten Arbeitstakts vorzunehmen ist, d.h, ob so vorgegangen werden soll, wie im Zusammenhang mit der Figur 4 beschrieben oder so wie im Zusammenhang mit der Figur 7 beschrieben.

In einer alternativen Ausführungsform werden in dem dritten Arbeitstakt das Ventil 30 und das Ventil 17 abgesperrt oder geschlossen werden oder geschlossen gehalten werden und der Druck in dem Austauschgefäß wird ausgansseitig auf einen zuvor gemessenen Druck gebracht, wobei der zuvor gemessene Druck dem niedrigeren der Drücke in dem Austauschgefäß während des ersten Arbeitstakt und während des zweiten Arbeitstakt entspricht.

D.h. entsprechend der im Zusammenhang mit den Figuren 2 und 3 beschriebenen Situation, bei der in dem Austauschgefäß während des zweiten Arbeitstakts ein höherer Druck als während des ersten Arbeitstakts herrscht, wird in dem dritten Arbeitstakt der Abfluss aus dem Teil des Dialysierflüssigkeitskreislaufs, der in dem ersten Arbeitstakt verbunden war, abgesperrt, das heißt das Ventil 16 wird abgesperrt oder geschlossen oder geschlossen gehalten. Weiterhin wird der Abfluss aus dem Teil des Dialysierflüssigkeitskreislaufs, der in dem zweiten Arbeitstakt verbunden war, geöffnet, das heißt das Ventil 12 wird geöffnet oder offen gehalten. Durch entsprechendes Ansteuern einer (nicht in der Figur 1 dargestellten) druckgesteuerten Pumpe in der zweiten Abflussleitung 11 stellt sich während des dritten Arbeitstakts in dem Austauschgefäß 32 ein Druck ein, der dem Druck während des ersten Arbeitstakts entspricht, also des Arbeitstakts, in dem der niedrigere Druck in dem Austauschgefäß herrschte. Anstatt einer Pumpe in der Abflussleitung könnte man auch einen vorhandenen hydrostatischen Druck nutzen.

Der dritte Arbeitstakt schließt sich in der zeitlichen Abfolge der Arbeitstakte an den zweiten Arbeitstakt an.

Entsprechend der im Zusammenhang mit den Figuren 5 und 6 beschriebenen Situation, bei der in dem Austauschgefäß während des ersten Arbeitstakts ein höherer Druck als während des zweiten Arbeitstakts herrscht, wird in dem dritten Arbeitstakt der Abfluss aus dem Teil des Dialysierflüssigkeitskreislaufs, der in dem zweiten Arbeitstakt verbunden war, abgesperrt, das heißt das Ventil 12 wird abgesperrt oder geschlossen oder geschlossen gehalten. Weiterhin wird der Abfluss aus dem Teil des Dialysierflüssigkeitskreislaufs, der in dem ersten Arbeitstakt verbunden war, geöffnet, das heißt das Ventil 16 wird geöffnet oder offen gehalten. Durch entsprechendes Ansteuern einer (nicht in der Figur 1 dargestellten) druckgesteuerten Pumpe in der ersten Abflussleitung 26 stellt sich während des dritten Arbeitstakts in dem Austauschgefäß 32 ein Druck ein, der dem Druck in während des zweiten Arbeitstakts entspricht, also des Arbeitstakts, in dem der niedrigere Druck in dem Austauschgefäß herrschte. Anstatt einer Pumpe in der Abflussleitung könnte auch ein vorhandener hydrostatischer Druck genutzt werden.

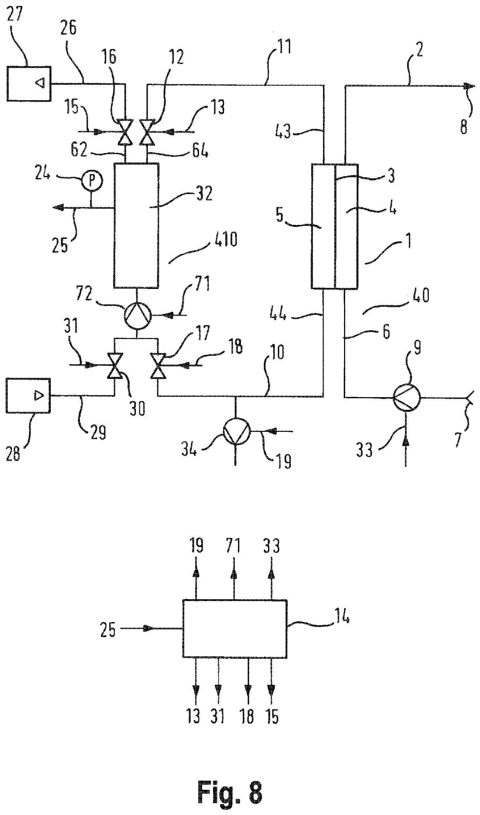

Figur 8 zeigt eine alternative Ausführungsform einer Hämodialysevorrichtung mit einer Bilanziervorrichtung 410. Die in der Figur 8 schematisch dargestellte Bilanziervorrichtung 410 entspricht im Wesentlichen der in der Figur 1 dargestellten Bilanziervorrichtung 41, wobei gleiche oder entsprechende Elemente mit gleichen Bezugszeichen versehen sind. Ein Unterschied zu der in der Figur 1 dargestellten Bilanziervorrichtung 41 besteht darin, dass an Stelle der Dialysierflüssigkeitspumpen 20 und 22 eine einzige Dialysierflüssigkeitspumpe 72 vorgesehen ist, die sowohl während des ersten als auch während des zweiten Arbeitstakts Dialysierflüssigkeit in das Austauschgefäß fördern kann. Die Dialysierflüssiglkeitspumpe 72 ist über eine Steuerleitung 71 mit der Steuereinheit 14 verbunden und durch diese ansteuerbar. Ein Vorteil dieser Anordnung ist einerseits dadurch gegeben, dass durch die Verwendung nur einer Dilaysierflüssigkeitspumpe die Anzahl der Komponenten der Dialysevorrichtung reduziert werden kann. Ein weiterer Vorteil ergibt sich im Zusammenhang mit der Situation, in der sich die Druckverhältnisse während der Dialysebehandlung verschieben und während der Behandlung entschieden wird, ob so vorgegangen werden soll, wie im Zusammenhang mit der Figur 4 beschrieben oder so vorgegangen werden soll, wie im Zusammenhang mit der Figur 7 beschrieben. In diesem Fall kann durch entsprechendes Ansteuern der Ventile zwischen diesen Situationen gewechselt werden, wobei eine einzige druckgesteuerte Pumpe in beiden Situationen angesteuert werden kann.

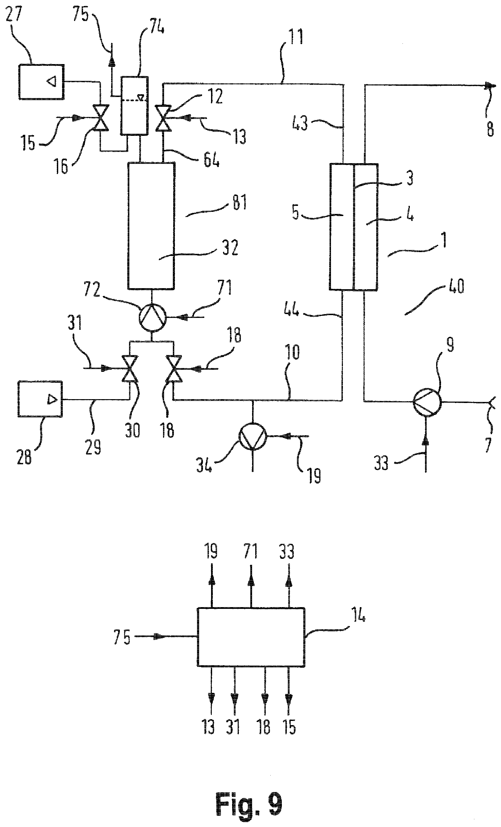

Figur 9 zeigt eine weitere Ausführungsform einer Hämodialysevorrichtung mit einer Bilanziervorrichtung 81. Die in Figur 9 schematisch dargestellte Hämodialysevorrichtung entspricht im Wesentlichen der im Zusammenhang mit der Figur 8 beschriebenen Vorrichtung, mit der Besonderheit, dass an Stelle oder als Druckmesseinrichtung 24 ein Ausgleichsgefäß 74 vorgesehen ist, dass an seiner Unterseite durch eine flüssigkeitsführende Leitung mit dem Austauschgefäß verbunden ist. In einer Ausführungsform ist das Ausgleichgefäß zusätzlich mit der Abflussleitung 26 verbunden und stellt so eine Fluidverbindung zwischen dem Austauschgefäß 32 und dem Abfluss 27. Nach oben hin ist das Ausgleichsgefäß 74 verschlossen. In dem Ausgleichsgefäß befindet sich eine Gasblase, die in Abhängigkeit von den Druckverhältnissen in dem Austauschgefäß 32 komprimierbar ist. Das Ausgleichsgefäß 74 weist weiterhin einen Pegelsensor zum Bestimmen des Flüssigkeitspegels auf, der in ein elektrisches Signal umgewandelt und durch die Datenleitung 75 an die Steuereinheit weitergegeben werden kann. Der Flüssigkeitspegel ersetzt oder entspricht einem Druck in dem Austauschgefäß und die Kombination des Austauschgefäßes mit dem Pegelsensor entspricht oder ersetzt den Druckmesser 24.

Dem Fachmann ist unmittelbar ersichtlich dass die Kombination eines Ausgleichsgefäßes mit einem Pegelsensor auch im Zusammenhang mit den anderen zuvor beschriebenen Ausführungsformen verwendet werden kann.

In alternativen, illustrativen, nicht erfindungsgemäßen Ausführungsformen kann auf einen dritten Arbeitstakt verzichtet werden, wenn durch das Ansteuern der während des ersten Arbeitstakts im ersten Dialysierflüssigkeitskreislauf befindlichen Dialysierflüssigkeitspumpe 21 und/ oder der während des zweiten Arbeitstakts im zweiten Dialysierflüssigkeitskreislauf befindlichen Dialysierflüssigkeitspumpe 22 sichergestellt wird, das während des ersten Arbeitstakts der Druck in dem Austauschgefäß 32 im Wesentlichen gleich dem Druck in dem zweiten Arbeitstakt ist, wobei insbesondere zum Ende des ersten Arbeitstakts der Druck in dem Austauschgefäß 32 gleich dem Druck in dem zweiten Austauschgefäß ist. So kann der Druck in dem Austauschgefäß in demjenigen Arbeitstakt, in dem der höhere Druck in dem Austauschgefäß 32 herrscht, zum Ende des Arbeitstakts durch entsprechendes ansteuern der Dialysierflüssigkeitspumpe, die in diesem Arbeitstakt mit dem Austauschgefäß 32 in einer fluidführenden Verbindung steht, abgesenkt werden.

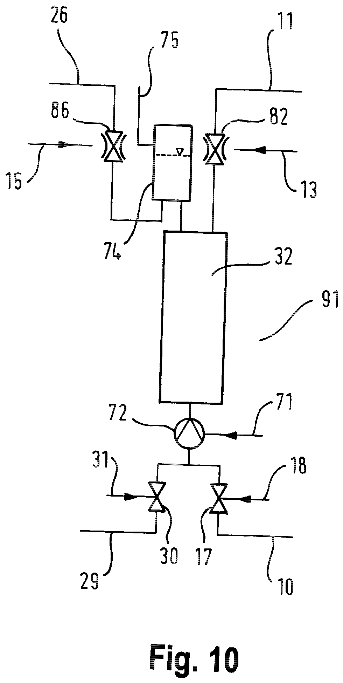

Figur 10 zeigt eine Weitergestaltung der im Zusammenhang mit der Figur 9 beschriebenen Bilanziervorrichtung. Mit entsprechenden Bezugszeichen versehene Elemente entsprechen denen der Figur 9, auf die Bezug genommen wird an Stelle einer Wiederholung. Die Weiterbildung der im Zusammenhang mit der Figur 9 beschriebenen Bilanziervorrichtung liegt darin, dass an Stelle der Ventile 16 und 12 in den Abflussleitungen 26 und 11, regelbare Drosselventile 86 und 82 in den Abflussleitungen 26 und 11 vorgesehen sind. Zusätzlich zu der Funktion eines Absperrorgans der Abflussleitungen 26 und 11 ermöglichen die regelbaren Drosselventile 86 und 82 eine Regulierung des Flusswiderstandes wenn der Fluss durch die Leitungen 26 und 11 nicht abgesperrt ist. So ermöglichen die regelbaren Drosselventile 86, 82 über eine Regelung des Flusswiderstands in den Leitungen 26 und 11 eine Regulierung des Drucks in dem Ausgleichgefäß 32. Auf diese Weise kann der in dem Austauschgefäß gemessene Druck verwendet werden, um das Drosselventil 86 sowie das Drosselventil 82 so anzusteuern, dass mindestens in einem Teil des ersten Arbeitstakts der Druck in der Austauschgefäß 32 im Wesentlichen gleich dem Druck in der Austauschgefäß 32 in mindestens in einem Teil des zweiten Arbeitstakts ist, wobei diese Bedingung vorzugsweise zum Ende des jeweiligen Arbeitstakts erfüllt sein soll. In dieser nicht beanspruchten

### Ausführungsform ist ein dritter

Arbeitstakt nicht erforderlich. Obwohl Ausführungsform der Figur 10 ein Austauschgefäß als einen Druckaufnehmer sowie eine Dialysierflüssigkeitspumpe, die in dem ersten sowie in dem zweiten Arbeitstakt verwendet werden kann zeigt, ist die in der Figur 10 dargestellte Weiterbildung darauf nicht beschränkt. So können auch andere Druckaufnehmer entsprechend der Darstellung der Figuren 1 und 8 verwendet werden oder eine Konfiguration, in der verschiedene Pumpen für den ersten sowie den zweiten Arbeitstakt vorgesehen sind, entsprechend der im Zusammenhang mit der Figur 1 beschriebenen Konfiguration. Ein dritter Arbeitstakt ist bei dem Betrieb der in der Figur 10 beschriebenen Bilanziervorrichtung nicht erforderlich, solange der Druck in dem Austauschgefäß 32 mit Hilfe regelbaren Drosselventile 86 und 82 so eingestellt wird, das gegen Ende des ersten Arbeitstakts derselbe Druck in dem Austauschgefäß 32 herrscht, wie gegen Ende des zweiten Arbeitstakts. Dazu können an Stelle der regelbaren Drosselventile 86, 82 auch Drosseln vorgesehen sein zusätzlich zu den Ventilen in den Absperrleitungen 11 und 26 vorgesehen sind und die eine Drosselfunktion unabhängig von einer Funktion eines Absperrorgans erfüllen. Der Druck in dem Austauschgefäß 32 kann auch durch einen kombinierten Einsatz der Drosselventile 86, 82 und der einen oder mehreren druckgesteuerten Pumpen in der oder den Zuleitungen zu dem Austauschgefäß 32 gesteuert werden, wobei der Druckausgleich zwischen dem ersten und zweiten Arbeitstakt so erfolgt, wie oben beschrieben.

## Patentansprüche

1. Bilanziervorrichtung (41, 410, 81, 91) zum Bilanzieren zwischen einem Zufluss (43) in eine Blutbehandlungseinheit (1) einer medizinischen Behandlungsvorrichtung und einem Abfluss (44) aus der Blutbehandlungseinheit (1), mit einer Bilanziereinheit die mindestens ein Austauschgefäß (32) aufweist,
einer zu dem Austauschgefäß führenden ersten Zuleitung (29) zum Zuführen von Flüssigkeit aus einer Flüssigkeitsquelle (28) in das Austauschgefäß (32), und einer von dem Austauschgefäß (32) abgehenden ersten Abflussleitung (26) zum Abführen der Flüssigkeit aus dem Austauschgefäß (32) in einen Ablauf (27),
einer von dem Austauschgefäß (32) abgehenden zweiten Abflussleitung (11) zum Abführen von Flüssigkeit aus dem Austauschgefäß (32) in die Behandlungseinheit (1) und einer zu dem Austauschgefäß (32) führenden zweiten Zuleitung (10) zum Zuführen von Flüssigkeit aus der Behandlungseinheit (1) in das Austauschgefäß (32),
Mitteln zum Fördern von Flüssigkeit (21, 22, 72) in das und/ oder aus dem Austauschgefäß und Mittel zum Unterbrechen des Zuflusses von Flüssigkeit in das Austauschgefäß durch die erste und durch die zweite Zuleitung (30, 17) und/ oder zum Unterbrechen des Abflusses von Flüssigkeit aus dem Austauschgefäß durch die erste und durch die zweite Abflussleitung (16, 12),
einer Steuereinheit (14) zum Ansteuern der Mittel zum Fördern von Flüssigkeit (21, 22, 72) und der Mittel zum Unterbrechen des Zuflusses und/ oder Abflusses von Flüssigkeit (16, 12, 30, 17), wobei die Steuereinheit (14) derart ausgebildet ist,
dass in einem ersten Arbeitstakt von aufeinanderfolgenden Arbeitstakten ein erster Fluidkreislauf gebildet wird, in dem verbrauchte Flüssigkeit aus dem Austauschgefäß (32) in den Ablauf (27) strömt und durch frische Flüssigkeit aus der Flüssigkeitsquelle (28) ersetzt wird und in einem zweiten Arbeitstakt der aufeinanderfolgenden Arbeitstakte ein zweiter Fluidkreislauf gebildet wird, in dem frische Flüssigkeit aus dem Austauschgefäß (32) in die Behandlungseinheit (1) strömt und durch verbrauchte Flüssigkeit aus der Behandlungseinheit (1) ersetzt wird,
und **gekennzeichnet durch** einen mit der Steuereinheit (14) verbundenen Druckaufnehmer (24) zum Aufnehmen des Drucks in dem Austauschgefäß während des ersten, des zweiten sowie während eines dritten Arbeitstakts,
und wobei die Steuereinheit (14) weiterhin ausgebildet ist, in dem dritten Arbeitstakt die erste und die zweite Abflussleitung abzusperren, und der Druck in dem Austauschgefäß auf einen zuvor gemessenen Druck gebracht wird, wobei der zuvor gemessene Druck dem höheren der Drücke in dem Austauschgefäß während des ersten Arbeitstakts und während des zweiten Arbeitstakts entspricht, oder in dem dritten Arbeitstakt die erste und die zweite Zuleitung abzusperren, und der Druck in dem Austauschgefäß wird ausgansseitig auf einen zuvor gemessenen Druck gebracht, wobei der zuvor gemessene Druck dem niedrigeren der Drücke in dem Austauschgefäß während des ersten Arbeitstakt und während des zweiten Arbeitstakt entspricht.

2. Bilanziervorrichtung (81, 91) nach Anspruch 1 wobei das Mittel zum Fördern von Flüssigkeit (72) in die und/ oder aus dem Austauschgefäß derart mit dem ersten und zweiten Zufluss (29, 10) oder mit dem ersten und zweiten Abfluss durch eine fluidführende Leitung verbindbar ist, dass das Mittel zum Fördern von Flüssigkeit (72) Flüssigkeit während des ersten Arbeitstakts und während des zweiten Arbeitstakts fördern kann.

3. Bilanziervorrichtung (41, 410, 81, 91) nach einem der vorangehenden Ansprüche, wobei die Steuereinheit (14) eingerichtet ist, die Mittel zum Fördern von Flüssigkeit (21, 22, 72) in die und/ oder aus dem Austauschgefäß (32) unter Verwendung eines Messsignals des Druckaufnehmers (14) anzusteuern.

4. Bilanziervorrichtung (41, 410, 81, 91) nach Anspruch 3, wobei das Mittel zum Fördern von Flüssigkeit (21, 22, 72) in die und/ oder aus dem Austauschgefäß (32) eine druckgesteuerte Pumpe ist.

5. Bilanziervorrichtung (41, 410, 81, 91) nach Anspruch 4, wobei die druckgesteuerte Pumpe (21, 22, 72) eine Impellerpumpe oder eine Membranpumpe ist.

6. Bilanziervorrichtung (81, 91) nach einem der vorangehenden Ansprüche, wobei der Druckaufnehmer als gasgefülltes Ausgleichsgefäß (74) ausgeführt ist, das in Fluidverbindung mit dem Austauschgefäß (32) steht und wobei ein Messmittel zum Bestimmen des Flüssigkeitsniveaus in dem Ausgleichsgefäß vorgesehen ist, und wobei das Flüssigkeitsniveau ein Maß für den Duck in dem Austauschgefäß angibt.

7. Bilanziervorrichtung (41, 410, 81, 91) nach einem der vorangehenden Ansprüche, wobei das Austauschgefäß bei einer Änderung des Drucks in dem Austauschgefäß nicht volumenkonstant ist.

8. Bilanziervorrichtung (41, 410, 81, 91) nach einem der vorangehenden Ansprüche, wobei das Austauschgefäß als Einwegartikel oder als Teil eines Einwegartikels ausgeführt ist.

9. Medizinische Behandlungsvorrichtung mit einer Bilanziervorrichtung (41, 410, 81, 91) nach einem der vorangehenden Ansprüche.

10. Medizinische Behandlungsvorrichtung nach Anspruch 9, ausgebildet als Dialysevorrichtung.

## Claims

1. Balancing device (41, 410, 81, 91) for balancing between an inflow (43) into a blood treatment unit (1) of a medical treatment device and an outflow (44) out of the blood treatment unit (1), with a balancing unit which has at least one exchange vessel (32),
a first inlet line (29) leading to the exchange vessel, for feeding liquid from a liquid source (28) into the exchange vessel (32), and a first outflow line (26) issuing from the exchange vessel (32), for discharging the liquid from the exchange vessel (32) into a drain (27),
a second outflow line (11) issuing from the exchange vessel (32), for discharging liquid from the exchange vessel (32) into the treatment unit (1), and a second inlet line (10) leading to the exchange vessel (32), for feeding liquid from the treatment unit (1) into the exchange vessel (32),
means (21, 22, 72) for conveying liquid into and/or out of the exchange vessel, and means (30, 17) for interrupting the inflow of liquid into the exchange vessel through the first and through the second inlet line and/or means (16, 12) for interrupting the outflow of liquid out of the exchange vessel through the first and through the second outflow line,
a control unit (14) for controlling the means (21, 22, 72) for conveying liquid and the means (16, 12, 30, 17) for interrupting the inflow and/or outflow of liquid, wherein the control unit (14) is designed in such a way that,
in a first work cycle of successive work cycles, a first fluid circulation is formed in which spent liquid from the exchange vessel (32) flows into the drain (27) and is replaced by fresh liquid from the liquid source (28) and, in a second work cycle of the successive work cycles, a second fluid circulation is formed in which fresh liquid from the exchange vessel (32) flows into the treatment unit (1) and is replaced by spent liquid from the treatment unit (1),
and **characterized by** a pressure detector (24), connected to the control unit (14), for detecting the pressure in the exchange vessel during the first work cycle, the second work cycle and a third work cycle,
and wherein the control unit (14) is further designed to shut off the first and the second outflow line in the third work cycle, and the pressure in the exchange vessel is brought to a previously measured pressure, wherein the previously measured pressure corresponds to the higher of the pressures in the exchange vessel during the first work cycle and during the second work cycle, or to shut off the first and the second inlet line in the third work cycle, and the pressure in the exchange vessel is brought at the outlet side to a previously measured pressure, wherein the previously measured pressure corresponds to the lower of the pressures in the exchange vessel during the first work cycle and during the second work cycle.

2. Balancing device (81, 91) according to Claim 1, wherein the means (72) for conveying liquid into and/or out of the exchange vessel can be connected to the first and second inlets (29, 10) or to the first and second outflows through a liquid-carrying line, in such a way that the means (72) for conveying liquid can convey liquid during the first work cycle and during the second work cycle.

3. Balancing device (41, 410, 81, 91) according to one of the preceding claims, wherein the control unit (14) is set up to control the means (21, 22, 72) for conveying liquid into and/or out of the exchange vessel (32) using a measurement signal of the pressure detector (14).

4. Balancing device (41, 410, 81, 91) according to Claim 3, wherein the means (21, 22, 72) for conveying liquid into and/or out of the exchange vessel (32) is a pressure-controlled pump.

5. Balancing device (41, 410, 81, 91) according to Claim 4, wherein the pressure-controlled pump (21, 22, 72) is an impeller pump or a diaphragm pump.

6. Balancing device (81, 91) according to one of the preceding claims, wherein the pressure detector is designed as a gas-filled equalizing vessel (74) which is in fluidic connection with the exchange vessel (32), and wherein a measurement means is provided for determining the liquid level in the equalizing vessel, and wherein the liquid level is a measure of the pressure in the exchange vessel.

7. Balancing device (41, 410, 81, 91) according to one of the preceding claims, wherein the exchange vessel is not of constant volume when there is a change in the pressure in the exchange vessel.

8. Balancing device (41, 410, 81, 91) according to one of the preceding claims, wherein the exchange vessel is designed as a disposable item or as part of a disposable item.

9. Medical treatment device having a balancing device (41, 410, 81, 91) according to one of the preceding claims.

10. Medical treatment device according to Claim 9, designed as a dialysis device.

## Revendications

1. Dispositif d'équilibrage (41, 410, 81, 91) destiné à effectuer un équilibrage entre un flux d'entrée (43) arrivant dans une unité de traitement de sang (1) d'un dispositif de traitement médical et un flux de sortie (44) de l'unité de traitement de sang (1), ledit dispositif d'équilibrage comprenant
une unité d'équilibrage qui comporte au moins un récipient d'échange (32),
une première conduite d'amenée (29) menant au récipient d'échange et destinée à amener un liquide d'une source de liquide (28) jusque dans le récipient d'échange (32), et une première conduite de flux de sortie (26) partant du récipient d'échange (32) et destinée à évacuer le liquide du récipient d'échange (32) jusque dans un moyen d'évacuation (27),
une deuxième conduite de flux de sortie (11) partant du récipient d'échange (32) et destinée à évacuer un liquide du récipient d'échange (32) jusque dans l'unité de traitement (1) et une deuxième conduite d'amenée (10) menant au récipient d'échange (32) et destinée à amener un liquide de l'unité de traitement (1) jusque dans le récipient d'échange (32),
des moyens de transport de liquide (21, 22, 72) jusque dans et/ou depuis le récipient d'échange et des moyens d'interruption du flux d'entrée de liquide dans le récipient d'échange par le biais des première et deuxième conduites d'amenée (30, 17) et/ou d'interruption du flux de sortie de liquide du récipient d'échange par le biais des première et deuxième conduites de flux de sortie (16, 12),
une unité de commande (14) destinée à commander les moyens de transport de liquide (21, 22, 72) et les moyens d'interruption du flux d'entrée et/ou du flux de sortie de liquide (16, 12, 30, 17), l'unité de commande (14) étant conçue de telle sorte que
dans un premier cycle de travail de cycles de travail successifs est formé un premier circuit de fluide, dans lequel le liquide usé s'écoule du récipient d'échange (32) jusque dans le moyen de sortie (27) et est remplacé par du liquide frais provenant de la source de liquide (28) et dans un deuxième cycle de travail des cycles de travail successifs est formé un deuxième circuit de fluide dans lequel du liquide frais s'écoule du récipient d'échange (32) jusque dans l'unité de traitement (1) et est remplacé par du liquide usé provenant de l'unité de traitement (1),
et **caractérisé par** un capteur de pression (24) relié à l'unité de commande (14) et destiné à recevoir la pression dans le récipient d'échange pendant le premier, le deuxième et un troisième cycle de travail, et l'unité de commande (14) étant en outre conçue pour fermer les première et deuxième conduites de flux de sortie dans le troisième cycle de travail, et la pression dans le récipient d'échange étant amenée à une pression précédemment mesurée, la pression précédemment mesurée correspondant à la plus élevée des pressions dans le récipient d'échange pendant le premier cycle de travail et pendant le deuxième cycle de travail, ou pour fermer les première et deuxième conduites d'amenée dans le troisième cycle de travail, et la pression dans le récipient d'échange étant amenée côté sortie à une pression précédemment mesurée, la pression précédemment mesurée correspondant à la plus faible des pressions dans le récipient d'échange pendant le premier cycle de travail et pendant le deuxième cycle de travail.

2. Dispositif d'équilibrage (81, 91) selon la revendication 1, le moyen de transport de liquide (72) jusque dans et/ou depuis le récipient d'échange pouvant être relié aux premier et deuxième flux d'entrée (29, 10) ou aux premier et deuxième flux de sortie par le biais d'une conduite de transport de fluide de telle sorte que le moyen de transport de liquide (72) puisse transporter du liquide pendant le premier cycle de travail et pendant le deuxième cycle de travail.

3. Dispositif d'équilibrage (41, 410, 81, 91) selon l'une des revendications précédentes, l'unité de commande (14) étant conçue pour commander les moyens de transport de liquide (21, 22, 72) jusque dans et/ou depuis le récipient d'échange (32) à l'aide d'un signal de mesure du capteur de pression (14).

4. Dispositif d'équilibrage (41, 410, 81, 91) selon la revendication 3, le moyen de transport de liquide (21, 22, 72) jusque dans et/ou depuis le récipient d'échange (32) étant une pompe commandée en pression.

5. Dispositif d'équilibrage (41, 410, 81, 91) selon la revendication 4, la pompe commandée en pression (21, 22, 72) étant une pompe à roue ou une pompe à membrane.

6. Dispositif d'équilibrage (81, 91) selon l'une des revendications précédentes, le capteur de pression étant réalisé sous la forme d'un récipient de compensation (74) rempli de gaz qui est en liaison fluidique avec le récipient d'échange (32) et un moyen de mesure étant prévu pour déterminer le niveau de liquide dans le récipient de compensation, et le niveau de liquide indiquant une mesure de la pression dans le récipient d'échange.

7. Dispositif d'équilibrage (41, 410, 81, 91) selon l'une des revendications précédentes, le volume du récipient d'échange n'étant pas constant lorsque la pression dans le récipient d'échange varie.

8. Dispositif d'équilibrage (41, 410, 81, 91) selon l'une des revendications précédentes, le récipient d'échange étant conçu comme un article à usage unique ou comme partie d'un article à usage unique.

9. Dispositif de traitement médical comprenant un dispositif d'équilibrage (41, 410, 81, 91) selon l'une des revendications précédentes.

10. Dispositif de traitement médical selon la revendication 9, conçu comme dispositif de dialyse.
